Europäisches Patentamt

(19) European Patent Office

Office européen des brevets

(11) **EP 0 304 279 B1**

(12) **EUROPEAN PATENT SPECIFICATION**

(45) Date of publication and mention
of the grant of the patent:
**05.11.1997 Bulletin 1997/45**

(51) Int. Cl.$^6$: **C07K 14/47**, A61K 38/04

(21) Application number: **88307608.5**

(22) Date of filing: **17.08.1988**

(54) **Peptide determinant associated with immunity**

Peptid-Determinante mit Einfluss auf die Immunität

Déterminant peptidique associé à l'immunité

(84) Designated Contracting States:
**AT BE CH DE ES FR GB GR IT LI LU NL SE**

(30) Priority: **17.08.1987 US 86694**

(43) Date of publication of application:
**22.02.1989 Bulletin 1989/08**

(60) Divisional application:
**97106788.9**

(73) Proprietor:
**THE BOARD OF TRUSTEES OF
THE LELAND STANFORD JUNIOR UNIVERSITY
Stanford California 94305 (US)**

(72) Inventors:
• **Steinman, Lawrence
Palo Alto, CA 94301 (US)**
• **Zamvil, Scott
Belmont, CA 94002 (US)**

(74) Representative:
**Harrison, David Christopher et al
MEWBURN ELLIS
York House
23 Kingsway
London WC2B 6HP (GB)**

(56) References cited:
• **ATURE, Vol. 324, 20 November 1986, pages 258-260; S.S. ZAMVIL et al.: "T-cell epitope of the autoantigen myelin basic protein that induces encephalomyelitis"**
• **J. Immunol., vol. 136(7), 2426-31 (1986)**
• **J. Immunol. vol. 136(7), 2426-31 (1986)**

Remarks:
• Divisional application 97106788.9 filed on 24/04/97.
• The file contains technical information submitted after the application was filed and not included in this specification

## Description

Modulation of immune response is achieved employing peptides which include a "motif" in the sequence. The motif defines sequences of an immunogen which can be used for modulating the immune response to an antigen. Specific motifs are avoided in relation to endogenous host proteins.

Mammalian species have evolved in association with pathogens, where the host has had to evolve mechanisms to protect itself for survival. The immune system, which is the primary protective mechanism, has had to achieve the ability to respond to a wide variety of life threatening situations, involving not only pathogens, but also endogenous diseases such as tumors and genetic defects. In developing protective mechanisms. the immune system has developed a series of different pathways, where the individual pathways may respond to different stimuli to protect the host. Many of the pathways of the immune system are associated with detection of a foreign antigenic substance, which may then stimulate B-cells or T-cells to prevent invading pathogens from proliferating and to remove toxic substances.

Despite the extraordinary spectrum of antigens with which the immune system must contend, the immune system must be able to distinguish between self and nonself. In many instances, there is a failure of the immune system resulting in attacks made on endogenous proteins and/or cells. Such autoimmune diseases may be extremely debilitating and can lead to death.

The fact that pathogens are able to invade a mammalian host and cause disease is evidence of the fact that while in most cases the immune system is able to prevent the infection from being mortal, the immune system must cope with the infection after it has become established. Since in many cases, diseases can be not only debilitating, but have permanent effects, there is substantial interest in preventing the establishment of a pathogen, such as a virus, bacteria, or other microorganism. Toward this end, vaccines have been developed which activate the immune system, so that upon invasion by the targeted pathogen, the immune system is able to respond rapidly and protect the host. There is also interest in modulating the immune system to allow for organ transplants, inhibit autoimmune diseases, and enhance the host response to neoplasia or other native cell diseased state. Therefore, there is substantial interest in developing techniques and compositions which will allow for improved protection against a variety of diseases.

### Relevant Literature

Steinman, et al., Nature (1977) 265:173-175, describes the regulation of autosensitization to encephalitogenic myelin basic protein by macrophage-associated insoluble antigen.

Steinman, et al., Neurology (1980) 30:755-759, describes a study involving soluble protein in evaluating cell tolerance to myelin basic protein. Jahnke, et al., Science (1985) 229:282-284, reports a comparison of sequence homology between certain viral proteins and proteins related to encephalomyelitis and neuritis. Zamvil, et al., Nature (1985) 317:355-358, describe induction of chronic relapsing paralysis and demyelination by T-cell clones specific for myelin basic protein. Zamvil, et al., Nature (1986) 324:258-260, and Zamvil, et al, J. Immunol (1987), 139: 1075-1079, describe a T-cell epitope of the autoantigen myelin basic protein that induces encephalomyelitis. Sriram, et al., Cellular Immunology (1983) 75:378-382, describe the effect of intravenous administration of mouse myelin basic protein coupled to syngeneic spleen cells to prevent the induction of experimental allergic encephalitis (EAE). See also Sette et al. Nature (1987) 328:395 who report sequences coming within the subject motif.

Price et al, J Immunol (1986), 136(7):2426-2431, used epitopes in a region of human and bovine MBP to raise four monoclonal antibodies, from which the conclusion was drawn that most, if not all, of the surface of a protein is immunogenic, rather than there being a single dominant epitope in the region 80-89 (as had been previously suggested).

### SUMMARY OF THE INVENTION

According to one aspect of the present invention there is provided a polypeptide which comprises a sequence of at least 9 amino acids from P89-101 of human myelin basic protein (hMBP) including the site of amino acids HFFK, which sequence is modified by not more than two non-conservative lesions.

According to a second aspect of the present invention there is provided a polypeptide which comprises a sequence of at least 9 amino acids from P89-101 of human myelin basic protein (hMBP) including the site of amino acids HFFK, which sequence is modified by not more than two lesions.

According to a third aspect of the present invention there is provided a polypeptide of not more than 15 amino acids, which comprises a sequence of at least 9 amino acids from P89-101 of human myelin basic protein (hMBP) including the site of amino acids HFFK, which sequence is optionally modified by not more than two non-conservative lesions.

According to a fourth aspect of the present invention there is provided a polypeptide of not more than 15 amino acids, which comprises a sequence of at least 9 amino acids from P89-101 of human myelin basic protein (hMBP) including the site of amino acids HFFK, which sequence is optionally modified by not more than two lesions.

According to a fifth aspect of the invention there is provided the use of a polypeptide of fewer than 60 amino acids which comprises a sequence of 9 amino acids from P89-101 of human myelin basic protein (hMBP) including the site of amino acids HFFK, the sequence optionally having up to two non-conservative lesions, in the manufacture of a medicament for tolerizing a mammalian host immune system comprising B cells and T cells to an immunogen of interest, wherein the immunogen is restricted by a transplantation antigen of said host.

According to a sixth aspect of the invention there is provided the use of a polypeptide of fewer than 60 amino acids which comprises a sequence of 9 amino acids from P89-101 of human myelin basic protein (hMBP) including the site of amino acids HFFK, the sequence optionally having up to two lesions, in the manufacture of a medicament for tolerizing a mammalian host immune system comprising B cells and T cells to an immunogen of interest, wherein the immunogen is restricted by a transplantation antigen of said host.

According to a seventh aspect of the present invention there is provided the use of a polypeptide of at least 9 and fewer than 60 amino acids including a sequence comprising the amino acids HFFK, in the manufacture of a medicament for tolerizing a mammalian host immune system comprising B cells and T cells to an immunogen of interest, wherein the immunogen is restricted by a transplantation antigen of said host.

According to an eighth aspect of the invention there is provided the use of a polypeptide comprising a sequence of 9-15 amino acids from hMBP and including the site of amino acids HFFK, which sequence is modified by not more than two non-conservative lesions, in the manufacture of a medicament for tolerizing a mammalian host immune system comprising B cells and T cells to an immunogen of interest, wherein the immunogen is restricted by a transplantation antigen of said host.

According to a ninth aspect of the invention there is provided the use of a polypeptide comprising a sequence of 9-15 amino acids from hMBP and including the site of amino acids HFFK, which sequence is modified by not more than two lesions, in the manufacture of a medicament for tolerizing a mammalian host immune system comprising B cells and T cells to an immunogen of interest, wherein the immunogen is restricted by a transplantation antigen of said host.

The amino acid sequence numbering used herein in relation to MBP is as given in Martenson, 1984, in Experimental Allergic Encephalomyelitis. A Useful Model for Multiple Sclerosis. Alvard, ed Alan Liss, NY (see infra under Experimental, Synthetic MBP Peptides); notwithstanding that other numbering systems may sometimes have been used, for example in Price et al (supra), where the corresponding amino acid sequence would be 87-99.

Novel oligopeptides and their use are described for modulating the immune system in relation to a particular immunogen. The oligopeptides comprise an amino acid sequence defined by a "motif" present in the immunogen of interest. The motif-containing oligopeptide will have at least about 9 amino acids and need not have more than about 15 amino acids of the sequence of the immunogen, usually having the defined motif sequence as other than the C-terminal sequence of the oligopeptide sequence. One or more sequences may be identified in the same immunogen having the defined motif sequence. Oligopeptides containing these sequences may be used in a single composition to be effective with a plurality of haplotypes. The immunogen motif sequence containing fragment may be used by itself, as part of a larger fragment, where the immunogen is joined to other than the wild type sequence, or may be joined covalently to other organic molecules, either proteinaceous or non-proteinaceous.

In referring to an epitope, the epitope will be the basic element or smallest unit of recognition by a receptor, particularly immunoglobulins and T-cell receptors, where the sequence may be contiguous or non-contiguous, and the amino acids essential to the receptor recognition may be contiguous and/or non-contiguous in the sequence. T-cell epitopes involve contiguous residues, while immunoglobulin epitopes are conformational determinants, and may be either contiguous or non-contiguous.

The immune response which is modulated is predicated upon a ternary complex, involving a cell having a transplantation antigen, a T-cell receptor restricted by the transplantation antigen, and a polypeptide which specifically binds to the combination of transplantation antigen and T-cell receptor. The transplantation antigens may be divided into two classes, Class I and Class II. Class I is relatively ubiquitous, found on most nucleated cells of a mammalian host, and has a polymorphic 45 Kd transmembrane protein which is non-covalently associated with a 12Kd non-polymorphic protein "$\beta_2$ microglobulin." By contrast, Class II transplantation antigens are restricted to relatively few sets of cells, primarily lymphocytes, macrophages, and dendritic cells, and comprise polymorphic $\alpha$- and $\beta$-chains. In the case of Class I transplantation antigens, one is primarily concerned with cellular aberrations or diseased states, such as viral infection, mycoplasma infection, neoplasia, or the like, or with organ transplants. The T-cells involved will normally cause the destruction of the aberrant cell.

By contrast, Class II transplantation antigens are concerned with activation of the cellular immune system, resulting in the expansion of cells involved with protection of the host against aberrant physiological states. The aberrant physiological states may be involved with pathogenic invasions, including viruses, bacteria, fungi, protista, toxins, or the like. In addition, the Class II cells may be involved with various autoimmune diseases, such as rheumatoid arthritis, systemic lupus erythematosus, diabetes, multiple sclerosis, etc., as well as those cellular aberrations or diseased states associated with Class I transplantation antigens.

Class I transplantation antigens are also involved in rejection of organ transplants. The immune system is able to recognize the foreign nature of the transplantation antigens present on the transplanted organ and attack the organ. In this situation, one does not wish to protect the host from the foreign cells, but rather diminish the subset of the immune system which is specific for attacking the organ transplant.

In many situations, there will also be interest in controlling lymphocyte response in vitro. These situations may involve specifically destroying cells infected with mycoplasma or virus, removing a particular subset of cells from a mixture of neoplastic and normal cells, expanding a particular subset or subsets of cells in a mixture, providing for conditioned medium for production of various lymphokines, such as IL-2, or the like. The in vitro system may involve whole blood, plasma, serum, cellular fractions, normal or immortalized cells, etc.

The motif sequence will be part of a sequence of an immunogen of interest, there usually being more than one partial sequence in the immunogen including the subject motif. The oligopeptide comprising the subject motif may be from any site of the immunogen sequence, that is N-terminal or C-terminal proximal or central, where the oligopeptide sequence will normally be substantially homologous with from 9 to 15 amino acids of the immunogen sequence, although longer sequences may also be employed. Usually the difference in homology will not be more than 2 non-conservative lesions, more usually not more than 2 lesions, which may be insertions, deletions, conservative or non-conservative substitutions.

Usually, the motif sequence present in the oligopeptide will be at other than the C-terminus of the oligopeptide, desirably being at the N-terminus and not closer to the C-terminus than the center of the sequence, where the second, third, or fourth amino acid of the motif (depending upon whether there are four or five amino acids in the motif) is the central amino acid.

The compositions of this invention will include usually at least one sequence of an immunogen of interest including the subject motif and may include two or more oligopeptide motif containing sequences of from about 9 to 15 amino acids present in the immunogen, depending upon the number of motifs present in the immunogen. Thus, if there are a plurality of motifs present in the immunogen, all or fewer than all of the sequences including the motifs may be employed in a single composition. Usually, there will be not more than 10 different motif comprising oligopeptides, more usually not more than about 6 different oligopeptides in the composition.

The oligopeptides employed in this invention which define motif containing regions and are not joined to heterologous sequences (that is sequences other than the natural sequence to which they are normally joined), will not include native myelin basic protein.

In preparing the subject compositions, one would select an immunogen of interest against which an immune response of a host is to be modulated. Thus, the immunogen of interest may be a protein associated with a pathogen. Pathogens include viruses, microorganisms, such as bacteria, fungi, protista, etc. The immunogen of interest may be a capsid protein, an enzyme protein, an envelope protein, a surface membrane protein, or any other protein which may naturally induce an immune response, which aids in the protection of the host from the pathogen. Other proteins of interest will include mammalian proteins, such as transplantation antigens, surface membrane proteins associated with blood type, or other cellular protein which may be recognized by a host immune system as foreign. A third group of oligopeptides will be associated with proteins endogenous to the host, associated with autoimmune diseases, where the oligopeptide may serve to tolerize the host to prevent immune attack against the endogenous protein or cell producing the endogenous protein. Other immunogens will also be of interest.

The particular protein of interest will be screened for the presence of the subject motif and one or more sequences including the motif selected. Where the haplotype of the intended recipient is known, one sequence may be preferred over another. However, where the haplotype is not known, or the composition may be administered to a number of different hosts, it will frequently be desirable to combine a number of the sequences as oligopeptides in the same composition. The oligopeptides may be present as the individual peptides, or may be joined together in a single sequence, with or without intervening bridges, where any bridges will be other than the naturally occurring intervening sequences of the immunogen. Desirably, any such sequence would have fewer than about 100 amino acids, more usually fewer than about 60 amino acids.

The subject oligopeptides may be modified in a variety of ways. For tolerization, the subject peptides may be conjugated to syngeneic spleen cells, or be linked to an innocuous immunogen to which the host has been previously immunized, such as tetanus toxoid, bovine serum albumin, etc. Adjuvants are normally avoided. Alternatively, the oligopeptide may be used for immunization (Steinman et al., Nature (1977) 265: 173-177; and Steinman et al., Neurology (1980) 30:755-759). To enhance the immune response against a plurality of immunogens, which may be associated with the same or different organism, as already indicated, the subject oligopeptides may be joined to oligopeptides sharing the same or different motif of the same immunogen or associated with an epitope of a different immunogen. The epitope may or may not include the subject motif. Thus, the resulting polypeptide will be able to activate, inactivate or tolerize the immune system to a plurality of immunogens, which are associated with the same or different organism. This approach may have particular advantages where vaccines are employed against a battery of diseases, such as the TORCH complex, various children's diseases, diseases endemic to particular locations, and the like.

The subject oligopeptides may not only be bonded to other oligopeptides, or proteins, but may be conjugated to a variety of non-proteinaceous products, particularly lipopolysaccharides, polysaccharides, lipids, glycerides, and the like. In many instances, the compounds may be inactivated toxins, provide for production of neutralizing antibodies or activate various cytotoxic T-cells toward a particular pathogen.

Transplantation antigens have polymorphic regions, where the individual alleles are associated with specific hosts. For the most part, the host will be diploid and heterozygous, so that each host will have two haplotypes, meaning that there will be two different copies of a particular transplantation antigen type from the same locus, unless the host is homozygous at that particular locus. Therefore, as to an individual host or a plurality of hosts, mixtures of oligopeptides will usually be employed.

The subject compositions find use in a variety of ways associated with the immune system. The subject compositions can be used as vaccines for activating the immune response to a particular pathogen or toxin. The subject compositions may be used to tolerize an individual, in the case of organ transplants, so that the rejection of the organ transplant or graft-v-host disease may be substantially reduced. The subject compositions may also be used to protect against autoimmune diseases, so that the immune system will be inhibited from attacking native proteins or host cells. The subject compositions may be used in the treatment of cancer. Other situations involving the immune system may also use the subject compositions with advantage.

Depending upon the particular application, the subject compositions may be administered in a variety of ways, by themselves or in conjunction with various additives. The subject compositions may be combined with various adjuvants, such as alum, BCG, oils, muramyl dipeptide. Alternatively, the subject compositions may be covalently bonded by any convenient linking group to an appropriate immunogen, such as tetanus toxoid, bovine gamma-globulin, keyhole limpet hemocyanin, etc. Various carriers may be employed which are physiologically acceptable, such as water, alcohol, saline, phosphate buffered saline, sugar, mineral oil, etc. Other additives may also be included, such as stabilizers, detergents, flavoring agents, thickeners, etc. The amount of active ingredient administered will vary widely depending upon the particular composition, the particular host, the number and frequency of administrations, the manner of administration, etc. Usually, there will be from about 0.01 to 10 $\mu$g/kg of host more usually from about 0.05 to 5 $\mu$g/kg of host, where the concentration may range from 10 $\mu$g/ml to 1 mg/ml.

The manner of administration may be varied widely, depending upon the formulation and nature of the active ingredient. Administration may be parenteral, intravascular, peritoneally, subcutaneous, oral, etc, may employ catheters, pumps, constant diffusion membranes, etc.

The subject compositions may also be used for diagnostic purposes for detecting T-cells which bind to the oligopeptide. Thus, the subject oligopeptide may be used in conjunction with a transplantation antigen and T-cells to determine the size of the T-cell population stimulated by a particular sequence. The subject peptide may also be used in competitive determinations to compare binding affinity of oligopeptides.

The subject compositions may be prepared in a variety of ways. The oligopeptides may be synthesized in accordance with conventional synthetic techniques, particularly automated synthesizers, or may be prepared by recombinant techniques, where an appropriate DNA sequence is devised for insertion into an expression cassette for expression in a microorganism host. Particularly, where the oligopeptide will be joined to one or more other oligopeptides, the recombinant techniques may be employed with advantage.

A large number of expression vectors are commercially available or have been described in the literature, where the expression vectors include transcriptional and translational initiation and termination regions separated by a polylinker having a plurality of unique restrictions sites. Sequences may be prepared coding for the oligopeptide or polypeptide of interest, which sequence may be inserted into the polylinker for expression in the host. If desired, the sequence may be prepared with a signal sequence, so that the product is secreted and processed to provide for the mature polypeptide.

Alternatively, the polypeptide may be covalently conjugated to another polypeptide or other molecule by providing for an appropriate functionality which can be used as a site for linking. For example, a cysteine may be used to react with an activated olefin, e.g., maleimide, so as to form a thioether. Alternatively, the oligopeptide may be modified at it's N-terminus to provide for a linking site, particularly where the oligopeptide is synthesized, which may include a variety of functional groups. If convenient, the synthesized oligopeptide may be conjugated while still bound to a support, followed by removing the conjugate from the support. Alternatively, a tyrosine may be used, which may be linked through a diazo group. Other conventional techniques may also be employed.

The subject oligopeptides may be derived from a wide variety of organisms including pathogens, such as retroviruses, such as the human retroviruses HTLV1-4, Pseudomonas, Bordatella pertussis, herpes simplex virus I and II. A number of pathogens are listed in U.S. Patent No. 4,208,479, which disclosure is incorporated herein by reference.

The following sequences may be included with advantage for tolerization in the polypeptides of the present invention. G-A-P-S; G-A-V-G; E-W-V-S; K-V-P-T; G-V-V-L-G; G-A-V-I-G; G-I-L-G; K-A-A-S (associated with $P_0$); Ac-A-S-Q-K-R; K-Y-L-A-T; G-I-L-D; R-F-F-G; K-I-F-K (associated with $P_1$); Ac-S-N-K-F-L; K-F-L-G; K-L-V-S; E-Y-M-K; G-L-A-T; R-V-I-I-S; K-M-V-V-E; R-I-Y-E- (associated with $P_2$); G-L-L-E; K-L-I-E; H-A-F-Q; G-A-V-R; K-W-L-G; K-F-V-G; R-M-Y-G; K-L-M-G (associated with PLP); and L-V-A-K; K-I-W-R; E-W-V-I-K; K-V-F-I-D; K-I-F-T; K-Y-I-A-E (associated with AChR). These sequences will normally be part of oligopeptide sequences of about 9 to 15 or more amino acids as described for the other motifs.

The following examples are offered by way of illustration and not by way of limitation.

EXPERIMENTAL

The determinant of myelin basic protein (MBP) P5-17 (Zamvil, et al., Nature, supra) contains a pattern P-S-Q-R-H-G-S-K-Y-L-A-T-A. Using the algorithm for the subject motif for predicting T-cell clones the epitope of the clone F1-28, a MBP-specific T-cell was isolated (Zamvil, et al., J. Exp. Med. (1985) 162: 2107), which clone recognizes the autoantigen myelin basic protein. A peptide corresponding to amino acids P35-47 containing two patterns GILD and RFFS was synthesized and shown to be stimulatory. The peptide determinant tested was G-I-L-D-S-I-G-R-F-F-S-G-D-R-G-A-P. The stimulatory epitope was shown with overlapping peptides to actually consist of L-D-S-I-G-R-F-F-S-G-D-R-G-A-P (Zamvil et al., Nature (1986) 324:258).

In another disease, myasthenia gravis, a T-cell epitope was discovered by using the subject algorithm to construct synthetic peptides of the acetylcholine receptor. The peptide AChR P215-232, D-T-P-Y-L-D-I-T-Y-H-F-V-M-Q-R-L-P-L was particularly stimulatory in a number of myasthenics. Other stimulatory peptides included 277-291 and 330-347 which followed the subject algorithm. Antigen-specific T-cell clones are isolated from peripheral blood lymphocytes (PBL), cultured in vitro with antigen and syngeneic irradiated PBL as antigen presenting cells (APL) (Cunningham et al., J. Gen. Virol. (1985) 66:249); Eckles et al., Nature (1981) 301:716).

To render autoimmunogenic peptides tolerigenic, these peptides may be conjugated to lymphocytes (Sriram, et al., 1983, supra) or by coupling the peptide to a carrier such as tetanus toxoid or bovine serum albumin, employing conventional linking groups (Herzenberg, et al., Ann. Rev. Imm. (1983) 1:609-632).

Synthetic MBP Peptides: Peptides corresponding to the amino acid sequences of rat (R) and bovine (B) MBP (Martenson, 1984, In Experimental Allergic Encephalomyelitis. A Useful model for multiple sclerosis. Alvard, ed. Alan Liss, N.Y.), were synthesized as described previously using solid phase techniques (Erickson and Merrifield, 1976, In The Proteins Vol. 2, Neurath, ed. Academic Press, NY, p 255). Peptides were separated from the various organic side products and the purity was determined by high pressure liquid phase column (Merck, Darmstadt, Germany) and by amino acid analysis. These peptides were not further purified since they all contained greater than 90% of the desired product.

The subject peptides described above were employed in the following test procedure:

Proliferation Assay: Proliferative responses were determined as described previously (Zamvil et al., Nature (1985) 317:355). 1x10^4 T-cells were cultured with 5x10^5 X-irradiated (3,000 rad) PL/J splenic APC in 0.2 ml of culture media in 96 well flat-bottomed microtiter plates (Falcon, 3072). Peptides were added to culture giving the final concentrations indicated. At 48 hours incubation, each well was pulsed with 1 $\mu$Ci $^3$H-thymidine and harvested 16 hours later. The mean c.p.m. thymidine incorporation was calulated for triplicate cultures. Standard deviations from replicate cultures were within 10% mean value.

P35-47 of the human myelin basic protein (MBP) comprising the RFFS motif was found to be stimulatory with mouse T-cells restricted by MHC I-$E_\alpha^u E_\beta^u$; P5-17 of the human myelin basic protein comprising the motif KYLAT was found to be stimulatory with mouse T-cells restricted by I-$A_\alpha^s A_\beta^u$ or I-$A_\alpha^u A_\beta^u$; and P89-101 of the human myelin basic protein comprising the motif HFFK was found to be stimulatory with mouse T-cells restricted by I-$A_\alpha^s A_\beta^s$.

The algorithm can be used to define what immunogenic part of an autoantigen shares sequence homology with pathogens. For example, in the case of MBP P35-47 shared with paramyxoviruses and influenza and MBP 89-101 shared with picornavirus, the critical sequence triggering the T-cell is shared with a pathogen.

It is evident from the above results, that sequences may be identified and employed as peptides for modulating the immune system in a variety of ways. As one illustration, a host may be tolerized against autoimmunogenic peptides by employing the subject peptides as tolerogens in a tolerizing manner. In other cases one may wish to immunize the host, so as to inhibit any further attack against endogenous cells or components. The method further provides for protection against various diseases resulting from vaccination, by eliminating any sequences which relate to stimulatory sequences found in endogenous proteins, particularly proteins involving the nervous system.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

**Claims**

1. A polypeptide which comprises a sequence of at least 9 amino acids from P89-101 of human myelin basic protein (hMBP) including the site of amino acids HFFK, which sequence is modified by not more than two non-conservative lesions (thereby excluding native hMBP protein).

2. A polypeptide which comprises a sequence of at least 9 amino acids from P89-101 of human myelin basic protein (hMBP) including the site of amino acids HFFK, which sequence is modified by not more than two lesions (thereby excluding native hMBP protein).

3. A polypeptide of not more than 15 amino acids, which comprises a sequence of at least 9 amino acids from P89-101 of human myelin basic protein (hMBP) including the site of amino acids HFFK, which sequence is optionally modified by not more than two non-conservative lesions.

4. A polypeptide of not more than 15 amino acids, which comprises a sequence of at least 9 amino acids from P89-101 of human myelin basic protein (hMBP) including the site of amino acids HFFK, which sequence is optionally modified by not more than two lesions.

5. A polypeptide according to claim 1 or claim 2, wherein the polypeptide has fewer than 100 amino acids.

6. A polypeptide according to claim 1 or claim 2, wherein the polypeptide has fewer than 60 amino acids.

7. A polypeptide according to claim 1 or claim 2 wherein the polypeptide has up to 15 amino acids.

8. A polypeptide according to any one of claims 1 to 7 for tolerizing a mammalian host immune system comprising B cells and T cells to an immunogen of interest, wherein said immunogen is restricted by a transplantation antigen of said host.

9. The use of a polypeptide according to any one of claims 1 to 7 in the manufacture of a medicament for tolerizing a mammalian host immune system comprising B cells and T cells to an immunogen of interest, wherein said immunogen is restricted by a transplantation antigen of said host.

10. The use of a polypeptide of fewer than 60 amino acids which comprises a sequence of 9 amino acids from P89-101 of human myelin basic protein (hMBP) including the site of amino acids HFFK, the sequence optionally having up to two non-conservative lesions, in the manufacture of a medicament for tolerizing a mammalian host immune system comprising B cells and T cells to an immunogen of interest, wherein the immunogen is restricted by a transplantation antigen of said host.

11. The use of a polypeptide of fewer than 60 amino acids which comprises a sequence of 9 amino acids from P89-101 of human myelin basic protein (hMBP) including the site of amino acids HFFK, the sequence optionally having up to two lesions, in the manufacture of a medicament for tolerizing a mammalian host immune system comprising B cells and T cells to an immunogen of interest, wherein the immunogen is restricted by a transplantation antigen of said host.

12. The use according to claim 10 or claim 11 wherein said sequence is from 9 to 15 amino acids in length.

13. The use of a polypeptide of at least 9 and fewer than 60 amino acids including a sequence comprising the amino acids HFFK, in the manufacture of a medicament for tolerizing a mammalian host immune system comprising B cells and T cells to an immunogen of interest, wherein the immunogen is restricted by a transplantation antigen of said host.

14. The use according to claim 13 wherein the polypeptide is not more than 15 amino acids in length.

15. The use of a polypeptide comprising a sequence of 9-15 amino acids from hMBP and including the site of amino acids HFFK, which sequence is modified by not more than two non-conservative lesions, in the manufacture of a medicament for tolerizing a mammalian host immune system comprising B cells and T cells to an immunogen of interest, wherein the immunogen is restricted by a transplantation antigen of said host.

16. The use of a polypeptide comprising a sequence of 9-15 amino acids from hMBP and including the site of amino acids HFFK, which sequence is modified by not more than two lesions, in the manufacture of a medicament for tolerizing a mammalian host immune system comprising B cells and T cells to an immunogen of interest, wherein the immunogen is restricted by a transplantation antigen of said host.

17. The use according to any one of claims 9 to 16, wherein said sequence is joined by chemical means to another epitope of hMBP.

18. A polypeptide according to any one of claims 1 to 8, wherein said sequence is joined by chemical means to another epitope of hMBP.

**Patentansprüche**

1. Ein Polypeptid, welches eine Sequenz von wenigstens 9 Aminosäuren von P89-101 von menschlichem Myelinbasisprotein (hMBP), das die Stelle aus Aminosäuren HFFK einschließt, umfaßt, dessen Sequenz durch nicht mehr als zwei nicht-konservative Läsionen modifiziert ist (und dadurch natives hMBP-Protein ausschließt).

2. Ein Polypeptid, welches eine Sequenz von wenigstens 9 Aminosäuren von P89-101 von menschlichem Myelinbasisprotein (hMBP), das die Stelle aus Aminosäuren HFFK einschließt, umfaßt, dessen Sequenz modifiziert ist durch nicht mehr als zwei Läsionen (und dadurch natives hMBP-Protein ausschließt).

3. Ein Polypeptid von nicht mehr als 15 Aminosäuren, welches eine Sequenz von wenigstens 9 Aminosäuren von P89-101 von menschlichem Myelinbasisprotein (hMBP), das die Stelle aus Aminosäuren HFFK einschließt, umfaßt, dessen Sequenz optional modifiziert ist durch nicht mehr als zwei nichtkonservative Läsionen.

4. Ein Polypeptid von nicht mehr als 15 Aminosäuren, welches eine Sequenz von wenigstens 9 Aminosäuren von P89-101 von menschlichem Basisprotein (hMBP), das die Stelle aus Aminosäuren HFFK einschließt, umfaßt, dessen Sequenz optional modifiziert ist durch nicht mehr als zwei Läsionen.

5. Ein Polypeptid nach Anspruch 1 oder Anspruch 2, wobei das Polypeptid weniger als 100 Aminosäuren aufweist.

6. Ein Polypeptid nach Anspruch 1 oder Anspruch 2, wobei das Polypeptid weniger als 60 Aminosäuren aufweist.

7. Ein Polypeptid nach Anspruch 1 oder 2, wobei das Polypeptid bis zu 15 Aminosäuren aufweist.

8. Ein Polypeptid nach irgendeinem der Ansprüche 1 bis 7, um ein Säugerwirtsimmunsystem, das B-Zellen und T-Zellen umfaßt, für ein interessierendes Immunogen tolerant zu machen, wobei das Immunogen beschränkt ist durch ein Transplantationsantigen des Wirts.

9. Die Verwendung eines Polypeptids nach einem der Ansprüche 1 bis 7 bei der Herstellung eines Medikaments, um ein Säugerwirtsimmunsystem, das B-Zellen und T-Zellen umfaßt, für ein interessierendes Immunogen tolerant zu machen, wobei das Immunogen beschränkt ist durch ein Transplantationsantigen des Wirts.

10. Die Verwendung eines Polypeptids von weniger als 60 Aminosäuren, welches eine Sequenz von 9 Aminosäuren von P89-101 von menschlichem Myelinbasisprotein (hBMP), das die Stelle aus Aminosäuren HFFK einschließt, umfaßt, wobei die Sequenz optional bis zu zwei nicht-konservative Läsionen aufweist, umfaßt, bei der Herstellung eines Medikaments, um ein Säugerwirtsimmunsystem, das B-Zellen und T-Zellen umfaßt, für ein interessierendes Immunogen tolerant zu machen, wobei das Immunogen durch ein Transplantationsantigen des Wirts beschränkt ist.

11. Die Verwendung eines Polypeptids von weniger als 60 Aminosäuren, das eine Sequenz von 9 Aminosäuren von P89-101 von menschlichem Myelinbasisprotein (hMBP), das die Stelle aus Aminosäuren HFFK einschließt, umfaßt, wobei die Sequenz optional bis zu zwei Läsionen aufweist, bei der Herstellung eines Medikaments, um ein Säugerwirtsimmunsystem, das B-Zellen und T-Zellen umfaßt, für ein interessierendes Immunogen tolerant zu machen, wobei das Immunogen durch ein Transplantationsantigen des Wirts beschränkt ist.

12. Die Verwendung nach Anspruch 10 oder Anspruch 11, wobei die Sequenz eine Länge von 9 bis 15 Aminosäuren aufweist.

13. Die Verwendung eines Polypeptids von wenigstens 9 und weniger als 60 Aminosäuren, einschließlich einer Sequenz, die die Aminosäuren HFFK umfaßt, bei der Herstellung eines Medikaments, um ein Säugerwirtsimmunsystem, das B-Zellen und T-Zellen umfaßt, für ein interessierendes Immunogen tolerant zu machen, wobei das Immunogen beschränkt ist durch ein Transplantationsantigen des Wirts.

14. Die Verwendung nach Anspruch 13, wobei das Polypeptid nicht länger als 15 Aminosäuren ist.

15. Die Verwendung eines Polypeptids, das eine Sequenz von 9 bis 15 Aminosäuren von hMBP umfaßt und die Stelle aus Aminosäuren HFFK einschließt, dessen Sequenz durch nicht mehr als zwei nicht-konservative Läsionen modifiziert ist, bei der Herstellung eines Medikaments, um ein Säugerwirtsimmunsystem, das B-Zellen und T-Zellen umfaßt, für ein interessierendes Immunogen tolerant zu machen, wobei das Immunogen beschränkt ist durch ein Transplantationsantigen des Wirts.

16. Die Verwendung eines Polypeptids, das eine Sequenz von 9 bis 15 Aminosäuren von hMBP umfaßt und die Stelle aus Aminosäuren HFFK einschließt, dessen Sequenz modifiziert ist durch nicht mehr als zwei Läsionen, bei der Herstellung eines Medikaments, um ein Säugerwirtsimmunsystem, welches B-Zellen und T-Zellen umfaßt, für ein interessierendes Immunogen tolerant zu machen, wobei das Immunogen beschränkt ist durch ein Transplantationsantigen des Wirts.

17. Die Verwendung nach einem der Ansprüche 9 bis 16, wobei die Sequenz durch chemische Mittel mit einem weiteren Epitop von hMBP verbunden ist.

18. Ein Polypeptid nach einem der Ansprüche 1 bis 8, wobei die Sequenz durch chemische Mittel mit einem weiteren Epitop von hMBP verbunden ist.

**Revendications**

1. Polypeptide qui comprend une séquence d'au moins 9 aminoacides provenant de P89-101 de la protéine basique de la myéline humaine (hMBP), y compris le site d'aminoacides HFFK, séquence qui est modifiée par pas plus de deux lésions non conservatrices (en excluant ainsi la protéine hMBP naturelle).

2. Polypeptide qui comprend une séquence d'au moins 9 aminoacides provenant de P89-101 de la protéine basique de la myéline humaine (hMBP), y compris le site d'aminoacides HFFK, séquence qui est modifiée par pas plus de deux lésions (en excluant ainsi la protéine hMBP naturelle).

3. Polypeptide de pas plus de 15 aminoacides, qui comprend une séquence d'au moins 9 aminoacides provenant de P89-101 de la protéine basique de la myéline humaine (hMBP), y compris le site d'aminoacides HFFK, séquence qui est facultativement modifiée par pas plus de deux lésions non conservatrices.

4. Polypeptide de pas plus de 15 aminoacides, qui comprend une séquence d'au moins 9 aminoacides provenant de P89-101 de la protéine basique de la myéline humaine (hMBP), y compris le site d'aminoacides HFFK, séquence qui est facultativement modifiée par pas plus de deux lésions.

5. Polypeptide suivant la revendication 1 ou la revendication 2, qui comprend moins de 100 aminoacides.

6. Polypeptide suivant la revendication 1 ou la revendication 2, qui comprend moins de 60 aminoacides.

7. Polypeptide suivant la revendication 1 ou la revendication 2, qui comprend jusqu'à 15 aminoacides.

8. Polypeptide suivant l'une quelconque des revendications 1 à 7, destiné à rendre tolérant le système immunitaire d'un mammifère hôte comprenant des lymphocytes B et des lymphocytes T vis-à-vis d'un agent immunogène intéressant, ledit agent immunogène étant soumis à une restriction par un antigène de transplantation dudit hôte.

9. Utilisation d'un polypeptide suivant l'une quelconque des revendications 1 à 7 dans la production d'un médicament destiné à rendre tolérant le système immunitaire d'un mammifère hôte comprenant des lymphocytes B et des lymphocytes T vis-à-vis d'un agent immunogène intéressant, ledit agent immunogène étant soumis à une restriction par un antigène de transplantation dudit hôte.

10. Utilisation d'un polypeptide de moins de 60 aminoacides qui comprend une séquence de 9 aminoacides provenant de P89-101 de la protéine basique de la myéline humaine (hMBP), y compris le site d'aminoacides HFFK, séquence ayant facultativement jusqu'à deux lésions non conservatrices, dans la production d'un médicament destiné à rendre tolérant le système immunitaire d'un mammifère hôte comprenant des lymphocytes B et des lymphocytes T vis-à-vis d'un agent immunogène intéressant, l'agent immunogène étant soumis à une restriction par un antigène de transplantation dudit hôte.

11. Utilisation d'un polypeptide de moins de 60 aminoacides qui comprend une séquence de 9 aminoacides provenant de P89-101 de la protéine basique de la myéline humaine (hMBP), y compris le site d'aminoacides HFFK, séquence ayant facultativement jusqu'à deux lésions, dans la production d'un médicament destiné à rendre tolérant le système immunitaire d'un mammifère hôte comprenant des lymphocytes B et des lymphocytes T vis-à-vis d'un agent immunogène intéressant, l'agent immunogène étant soumis à une restriction par un antigène de transplantation dudit hôte.

12. Utilisation suivant la revendication 10 ou la revendication 11, dans laquelle la séquence a une longueur de 9 à 15 aminoacides.

13. Utilisation d'un polypeptide d'au moins 9 aminoacides et de moins de 60 aminoacides comprenant une séquence renfermant les aminoacides HFFK, dans la production d'un médicament destiné à rendre tolérant le système immunitaire d'un mammifère hôte comprenant des lymphocytes B et des lymphocytes T vis-à-vis d'un agent immunogène intéressant, l'agent immunogène étant soumis à une restriction par un antigène de transplantation dudit hôte.

14. Utilisation suivant la revendication 13, dans laquelle le polypeptide a une longueur non supérieure à 15 aminoacides.

15. Utilisation d'un polypeptide comprenant une séquence de 9 à 15 aminoacides provenant de hMBP et comprenant le site d'aminoacides HFFK, séquence qui est modifiée par pas plus de deux lésions non conservatrices, dans la production d'un médicament destiné à rendre tolérant le système immunitaire d'un mammifère hôte comprenant des lymphocytes B et des lymphocytes T vis-à-vis d'un agent immunogène intéressant, l'agent immunogène étant soumis à une restriction par un antigène de transplantation dudit hôte.

16. Utilisation d'un polypeptide comprenant une séquence de 9 à 15 aminoacides provenant de hMBP et comprenant le site d'aminoacides HFFK, séquence qui est modifiée par pas plus de deux lésions dans la production d'un médicament destiné à rendre tolérant le système immunitaire d'un mammifère hôte comprenant des lymphocytes B et des lymphocytes T vis-à-vis d'un agent immunogène intéressant, l'agent immunogène étant soumis à une restriction par un antigène de transplantation dudit hôte.

17. Utilisation suivant l'une quelconque des revendications 9 à 16, dans laquelle la séquence est jointe par des moyens chimiques à un autre épitope de la hMBP.

18. Polypeptide suivant l'une quelconque des revendications 1 à 8, dans lequel la séquence est jointe par des moyens chimiques à un autre épitope de hMPB.